(19) Europäisches Patentamt
European Patent Office
Office européen des brevets

(11) Veröffentlichungsnummer: **0 266 503 B1**

(12) # EUROPÄISCHE PATENTSCHRIFT

(45) Veröffentlichungstag der Patentschrift: **12.12.90**

(51) Int. Cl.⁵: **C08F 220/32**, C08F 226/00, C08F 216/14, C12N 11/08

(21) Anmeldenummer: **87112389.9**

(22) Anmeldetag: **26.08.87**

(54) **Vernetzte Polymerisate und Verfahren zu ihrer Herstellung.**

(30) Priorität: **28.08.86 DE 3629177**

(43) Veröffentlichungstag der Anmeldung: **11.05.88 Patentblatt 88/19**

(45) Bekanntmachung des Hinweises auf die Patenterteilung: **12.12.90 Patentblatt 90/50**

(84) Benannte Vertragsstaaten: **AT BE CH DE FR GB IT LI NL SE**

(56) Entgegenhaltungen: **EP-A- 0 146 329**
**US-A- 3 012 010**

(73) Patentinhaber: **HOECHST AKTIENGESELLSCHAFT, Postfach 80 03 20, D-6230 Frankfurt am Main 80(DE)**

(72) Erfinder: **Mauz, Otto, Dr., Heidestrasse 21, D-6237 Liederbach(DE)**
Erfinder: **Noetzel, Siegfried, Dr., An den Römergärten 3, D-6233 Kelkheim (Taunus)(DE)**
Erfinder: **Sauber, Klaus, Dr., Friedrich Ebert-Str. 15, D-6231 Schwalbach (Taunus)(DE)**

**EP 0 266 503 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf vernetzte Polymerisate, die überwiegend in Form von kugelförmigen porösen Teilchen vorliegenden und auf Basis von Epoxydgruppen enthaltenden Monomeren, vernetzenden Monomeren und gegebenenfalls weiteren monoethylenisch ungesättigten Monomeren aufgebaut sind. Solche Polymerisate eignen sich sehr gut als Trägermaterialien für die Immobilisierung von biologisch aktiven Substanzen.

Es ist bekannt, biologisch aktive Substanzen, wie biespielsweise Enzyme, Antikörper, Antigene, Hormone, unter Erhalt ihrer Aktivität an polymere Trägermaterialien über kovalente Bindungen zu fixieren, um auf diesem Weg beispielsweise Enzyme zu stabilisieren, zu reinigen oder wasserunlöslich zu machen. Solchermaßen immobilisierte biologisch aktive Substanzen bieten erhebliche Vorteile gegenüber der löslichen Form: Zum einen ist die Abtrennbarkeit durch Sedimentation nach Beendigung einer Reaktion vereinfacht, zum anderen ist die Stabilität und Wiederverwendbarkeit der Präparate um ein Vielfaches erhöht.

Es ist auch bekannt, in ein hydrophiles Polymeres Oxirangruppen einzuführen, die dann zur Bindung eines biologisch wirksamen Stoffes verwendet werden können (vgl. DE-A 2 102 514). Unter den hydrophilen Polymeren sind auch solche genannt, die Acrylamidgruppen enthalten. Diesen Trägern fehlt jedoch die perlförmige Morphologie und die poröse Struktur. Dadurch sind sie beispielsweise für die Anwendung in einem Säulenverfahren nicht geeignet.

Als Trägersubstanzen wurden auch quellbare, vernetzte Perlpolymerisate beschriben, die durch Copolymerisation von reaktive Gruppen enthaltenden Monomeren, vernetzenden Monomeren und hydrophilen Monomeren erhalten werden (vgl. DE-B 2 237 316). Als reaktive Gruppe werden dabei die Halogenalkyl-, die Epoxyd-, die Carbonsäurechlorid-, Carbonsäureanhydrid-, Carbonsäureaxid-, Carbonsäurephenylester- und Hydroxamsäure-Gruppe offenbart. Diese Trägermaterialien haben jedoch eine Reihe von Nachteilen; so ist die Fixierung der biologisch aktiven Substanzen bei einigen von ihnen ziemlich langwierig; ihre Aktivität ist teilweise unbefriedigend und bei Verwendung der Anhydridvarianten kommt es außerdem zu Bildung von Carboxylgruppen, die unerwünscht sind.

Weiterhin sind Perlpolymerisate aus vernetzen Homo- oder Copolymerisaten des (Meth)acrylamides und/oder des Methylenbis(meth)acrylamids und gegebenenfalls weiteren radikalisch polymerisierbaren Comonomeren bekannt (vgl. DE-B 2 722 751). Diese Polymerisate eignen sich wegen des beispielsweise einpolymerisierten Glycidylmethacrylats oder Allylglycidylethers ebenfalls als Träger für biologische Wirkstoffe. Sie haben aber den Nachteil, daß bei ihrer Herstellung organische Lösungsmittel als Suspensionsmittel eingesetzt werden müssen und in Wasser nicht gearbeitet werden kann.

Bekannt ist auch, daß Glycidylester und Glycidylether enthaltende hydrophile Latexpartikel sich ebenfalls zur kovalenten Bindung von biologisch und/oder immunologisch aktiven Substanzen eigenen (vgl. EP-A 0 054 685). Diese Latexpartikel sind aber für viele Zwecke weniger geeignet als die z.B. in Säulen gut verwendbaren perlförmigen Polymerisate.

Ebenfalls bekannt sind Polymerisate, welche auch Glycidylacrylat, Glycidylmethacrylat und Allylglycidylether enthalten und mit Trivinylmonomeren vernetzt sind (vgl/ EP-A 0 146 329). Ihre Bindefähigkeit für Enzyme ist aber nur schwach ausgeprägt.

Aus der EP-A 0 058 767 ist ein Verfahren zur Herstellung oxirangruppenhaltiger, perlförmiger Polymerisate bekannt, bei welchem die Monomeren in einem besonderen Lösungsmittelgemisch polymerisiert werden. Aber auch hier muß die nachteilige inverse Perlpolymerisation angewandt werden.

Es bestand somit die Aufgabe, Polymerisate zur Immobilisierung von biologisch aktiven Substanzen wie z.B. Enzymen zu finden, welche sich auf eine sehr einfache Weise herstellen lassen und eine sehr gute Bindefähigkeit gegenüber biologisch aktiven Wirkstoffen aufweisen. Dies wurde dadurch erreichet, daß man vernetzte Polymerisate einsetzt, die aus Epoxydgruppen enthaltenden Monomeren, vernetzenden Monomern und ggfs. weiteren monoethylenisch ungesättigten Monomeren entstehen.

Die Erfindung betrifft somit ein vernetztes Polymiersat, bestehend im wesentlichen aus A) 1 bis 70 Gew.-% Einheiten, die sich von Glycidylacrylat, Glycidylmethacrylat, Allylglycidylether und/oder Vinylglycidylether ableiten, B) 99 bis 30 Gew.-% Einheiten, die sich von N,N'-Divinylethylenharnstoff und/oder N,N'-Divinylpropylenharnstoff ableiten, wobei die Summe der Einheiten stets 100 Gew-% ist und wobei die Polymerisatteilchen im wesentlichen kugelförmige Gestalt, eine mittlere Teilchengröße von 10 bis 600 µm und einen mittleren Porendurchmesser von 5 bis 1000 nm aufweisen.

Die Erfindung betrifft auch eine Verfahren zur Herstellung des genannten Polymerisats durch Copolymerisation der Monomeren in einem flüssigen Dispersionsmittel, welches unter den Polymerisationsbedingungen die Monomeren und das Polymerisat nicht löst, in Gegenwart eines radikalisch wirksamen Initiators und weiterer Hilfsstoffe sowie eines Stoffes, welcher sich in den Monomeren gut löst oder mit ihnen mischbar ist und im Dispersionsmittel praktisch unlöslich ist (Inertmittel), dadurch gekennzeichnet daß A') 1 bis 70 Gew.-%, bezogen das Monomerengemisch, Glycidylacrylat, Glycidylmethacrylat, Allylglycidylether undp/oder Vinylglycidylether und B') 99 bis 30 Gew.-%, bezogen auf das Monomerengemisch, N,N'-Divinylethylenharnstoff und/oder N,N'-Divinylpropylenharnstoff in Gegenwart von 50 bis 300 Gew.-%, bezogen auf die Summe der Monomeren, Inertmittel copolymerisiert werden.

Eine bevorzugte Ausführungsform des Verfahrens enthält als zusätzliche Komponente C') 0.1 bis 20 Gew.-%, bezogen auf das Monomerengemisch, Vinylacetat, Methylmethacrylat, Butylacrylat und/oder Styrol.

Die Erfindung betrifft schließlich auch die Verwendung der so erhaltene Polymerisate als Trägermaterialien zur Herstellung von trägergebundenen, biologisch aktiven Substanzen.

Das erfindungsgemäße Polymerisat besteht zu A) 1 bis 70 Gew.-%, vorzugsweise 5 bis 50 Gew.-%, insbesondere 10 bis 40 Gew.-% aus Einheiten, die sich von einem epoxydgruppenhaltigen Monomer A') ableiten, B) 30 bis 99 Gew.-%, vorzugsweise 40 bis 95 Gew.-%, insbesondere 45 bis 90 Gew.-% aus Einheiten, die sich von einem vernetzenden Monomer B') ableiten, und zusätzlich gegebenenfalls C) 0,1 bis 20 Gew.-%, vorzugsweise 0,1 bis 10 Gew.-% aus Einheiten, die sich von einem monoethylenisch ungesättigten, nicht hydrophilen und nichtvernetzenden Monomer C') ableiten. Die Gewichtsprozente sind jeweils auf das Gesamtpolymerisat bezogen.

Geeignete epoxydgruppenhaltige Monomere A') sind beispielsweise Glycidylacrylat, vorzugsweise Glycidylmethacrylat und Allylglycidylether, insbesondere Vinylglycidylether, allein oder im Gemisch.

Geeignete vernetzende Monomere B') sind z.B. N,N'-Divinylpropylenharnstoff, bevozugt jedoch N,N'-Divinylethylenharnstoff, allein oder im Gemisch.

Geeignete monoethylenisch ungesättigte, nicht hydrophile und nichtvernetzende Monomere C') sind z.B. Vinylacylate, Alkylacrylate, Alkylmethacrylate, Styrol, Styrolderivate, vorzugsweise Vinylacetat, Methylmethacrylat, Butylactylat und Styrol, allein oder im Gemisch.

Im erfindungsgemäßen Verfahren zur Herstellung des erfindungsgemäßen Polymerisats werden die Monomeren in Gegenwart eines radikalisch wirksamen Initiators und weiterer Hilfsstoffe im Suspensions-, Lösungs- oder Fällungspolymerisationsverfahren polymerisiert. Bevorzugt ist die Suspensionspolymerisation in Wasser als Suspensionsmittel bei einer Temperatur von 20 bis 120 °C, vorzugsweise von 25 bis 90 °C.

Als radikalisch wirksame Initiatoren kommen solche in Betracht, die in der Monomerphase gut und in Wasser schwer löslich sind. Beispiele hierfür sind organische Peroxide, wie Di-tert.-butylperoxid, Dibenzoylperoxid, Bis(o-Methylbenzoyl)peroxid, tert.-Butylhydroperoxyd, Cumolhydroperoxid, Di-isopropylperoxidicarbonat, Cyclohexanonperoxid oder aliphatische Azoverbindungen, wie α, α'-Azodiisobuttersäurenitril, Azobis-cyanvaleriansäure, 1,1'-Azo-cyclo-hexan-1,1'-dicarbonsäurenitril und Azodicarbonamid.

Bei der Suspensionspolymerisation werden Stabilisatoren und/oder Dispergierhilfsmittel verwendet wie beispielsweise Polyvinylpyrrolidon, Polyacrylamid, Polyvinylalkohol, Hydroxyethylcellulose.

Um eine möglichst hohe Porosität der Perlpolymerisate zu erreichen, werden dem Polymerisationssystem oder vorzugsweise den Monomeren bestimmte inerte, flüssige Komponente (Inertmittel) zugesetzt. Hierunter sollen solche Stoffe verstanden sein, in denen sich die Monomeren gut lösen oder mit ihnen mischbar, andererseits aber im Dispergiermittel praktisch unlöslich und damit mit diesem nicht mischbar sind. Nach ihrem Verhalten zu den entsprechenden Copolymeren kann man die Inertmittel in Quellungs- und/oder Fällungsmittel einteilen. Die Inertmittel nehmen an der Polymerisation nicht teil, werden jedoch von Polymerisat umhüllt und bei der Aufarbeitung wieder herausgelöst. Dadurch entstehen permanente Poren. Die Porengröße ist durch Art und Menge des Inertmittels beeinflußbar, hängt aber auch von der Menge an vernetzender Komponente ab.

Die bei der Polymerisation verwendeten Inertmittel, in denen die Monomeren gelöst werden, dürfen im vorliegenden Fall mit den ethylenischen Doppelbindungen und den Epoxydgruppen der Monomeren nicht reagieren.

Bevorzugte Inertmittel sind Pentanol, Heptylalkohol 2-Ethylhexanol, Nonylakohol, Decylalkohol, Laurylalkohol, Cyclohexanol und Oxoalkohole z.B. TCD Alkohol M

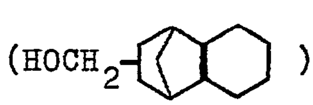

Die Inertmittel werden in einer Menge von 50 bis 300 Gew.-%, vorzugsweise 100 bis 250 Gew.-%, insbesondere 125 bis 200 Gew.-%, bezogen auf die Gesamtmenge der eingesetzten Monomeren, verwendet. Sie können allein oder im Gemisch eingesetzt werden.

Das erfindungsgemäße Verfahren wird zweckmäßigerweise in einem mit einer Rührvorrichtung versehenen Reaktionsgefäß durchgeführt. Die Teilchengröße des Perlpolymerisates wird in bekannter Weise durch die Rührgeschwindigkeit und das Phasenverhältnis eingestellt. Besonders vorteilhaft ist die Verwendung eines senkrecht stehenden, zylindrischen Gefäßes mit flachem Boden, das mit einem koaxial angebrachten Rührer versehen ist, dessen Welle bis fast auf den Gefäßboden reicht. Das Reaktionsgefäß ist vorzugsweise vakuumfest und kann mit Rückflußkühler, Zulauftrichter, Gaseinleitungsrohr und Temperaturmeßgerät versehen werden.

Die Beheizung und Kühlung des Gefäßes erfolgt im allgemeinen durch ein Flüssigkeitsbad, z.B. ein Ölbad oder Wasserbad.

Es ist vorteilhaft, das erfindungsgemäße Verfahren unter Ausschluß von Luftsauerstoff durchzuführen. Das Reaktionsgefäß wird daher vor Beginn mit einem inerten Gas, vorzugsweise Stickstoff, gespült.

Nach Beendigung der Polymerisationsreaktion werden die nicht umgesetzten Monomeren aus dem Reaktionsgefäß entfernt z.B. durch Verdampfen bei vermindertem Druck, vorzugsweise einem Druck von 13,33 bis 1999,8 Pa (0,1 bis 15 Torr). Nach der Entfernung der Restmonomeren wird das Dispersionsmittel vom festen Polymeren abgetrennt, z.B. durch Dekantieren, Filtrieren oder Absaugen des Überstandes. Anschließend wird das Polymerisat, falls erforderlich, mit leichtsiedenden organischen Lösungsmitteln, z.B. einesem Kohlenwasserstoff, einem niederen Alkohol oder Aceton gewaschen und schließlich getrocknet. Die Trocknung des Polymerisates erfolgt bei einer Temperatur von zumeist 20 bis 100 °C, vorzugsweise von 40 bis 80 °C; eine Trocknung unter vermindertem Druck ist dabei empfehlenswert.

Das erfindungsgemäße Perlpolymerisat besteht aus überwiegend kugelförmigen Teilchen, deren mittlere Teilchengröße im trocknen, ungequollenen Zustand 10 bis 600 µm, vorzugsweise 20 bis 400 µm beträgt und die vorzugsweise eine enge Teilchengrößenverteilung aufweisen. Das jeweilige O ptimum der Teilchengröße hängt dabei vor allem von dem speziellen Einsatzgebiet ab. Bei einem ohne Druck durchgeführten Säulenverfahren wird man beispielsweise die Teilchengröße innerhalb der vorstehend genannten Grenzen entsprechenden größer wählen können als bei einem Druckverfahren. Die Perlen des erfindungsgemäßen Perlpolymerisates sind überwiegend als makroporöse Perlen ausgebildet. Dies drückt sich in dem sich daraus ergebenden erfindungsgemäßen mittleren Porendurchmesser im Bereich von 5 bis 1000 nm, vorzugsweise 10 bis 800 nm aus.

Die Bestimmung des Porendurchmessers (Porenvolumens) erfolgt in der Weise, daß zunächst das Porenvolumen gemäß der Kapillardruckmethode (Quecksilberporosimetrie) bestimmt wird. Daneben ist eine Porengrößenbestimmung auch durch Rasterelektronenmikroskopie möglich.

Die erfindungsgemäßen Polymerisate eignen sich für die Immobilisierung von biologisch aktiven Substanzen durch Ausbildung einer kovalenten Bindung. Sie eignen sich aber auch, gegebenenfalls nach Inaktivierung der Epoxydgruppen, für andere Zwecke, wie z.B. die Affinitätschromatographie usw.

Unter dem Begriff "biologisch aktive Substanzen" seien die bekannten in vivo oder in vitro wirksamen natürlichen oder künstlich hergestellten Stoffe verstanden, beispielsweise Enzyme, Aktivatoren, Inhibitoren, Antigene, Antikörper, Vitamine, Hormone, Effektoren, Antibiotika, Proteine. Der Begriff Proteine umfaßt dabei auch Proteine mit bestimmten Nicht-Proteinsubstituenten wie Metallionen, Polysacchariden, Porphyringruppen, Adenindinucleotid, Ribonucleinsäure, Phospholipide etc. Auch Polypeptidfragmente, beispielsweise die aktiven Teile von Enzymmolekülen, fallen unter den Begriff "biologisch aktive Substanzen:.

Von den vorstehend genannten biologisch aktiven Substanzen sind die Enzyme bevorzugt. Beispiele für Enzyme sind Urease, Penicillinacylase, D-Aminosäureoxidase, Adenylesaminase, Alkohol-Dehydrogenase, Asparaginase, Carboxypeptidase, Chymotrypsin, Diphosphoesterase, α-Glucosidase, Glucose-Isomerase, Glucose-Oxidase, Glucose-6-phosphat-Dehydrogenase, Hexokinase, Invertase, β-Lactamase, Lactase, Lactat-Dehydrogenase, versch. Lectine, NAD-Kinase, Neuraminidase, Papain, Peroxidase, Phosphatasen (alkalisch und sauer), 5'-Phosphodiesterase, Pyruvat Kinase, Ribonuclease, Trypsin.

Beispiele für andere biologisch aktive Substanzen sind Hormone, wie Insulin und die verschiedensten Hypophysen-Hormone, Proteine der gamma-Globulinfraktion, z.B. Antihämophiliefaktor, die Blutgerinnungsfaktoren, spezielle Antikörper, z.B. Hepatitis-, Poliomyelitis-, Finnen-, Mumps-, Influenza- oder Kaninchenantikörper, Antigene, wie Hepatitis-, Poliomyelitis-, Finnen-, Mumps-, Influenza-oder Kaninchenantigene zur Reinigung oder Stimulierung geeigneter Antikörperreaktionen, wobei das Antigen (nach dem Unlöslichmachen) in der unlöslichen Form verbleibt und folglich nicht in den Körper eindringen und diesen schädigen kann, sowie allgemeine Körperproteine, wie Hämoglobin oder Albumin.

Die Bindung der biologisch aktiven Substanzen an das polymere Trägermaterial ist an sich bekannt und erfolgt allgemein so, daß das trockene Trägermaterial beispielsweise zu einer Enzymlösung gegeben wird, die mittels einer Pufferlösung, z.B. 1,5-molarer Kaliumphosphatlösung in Wasser, auf einem bestimmten pH-Wert eingestellt wird. Nach einer Fixierzeit, die 1 bis 72 Stunden betragen kann, wird bei einer bestimmten Temperatur (z.B. 23 °C) das Trägermaterial mit 1-molarer Kochsalzlösung und mit der Pufferlösung gut gewaschen. Am feuchten Trägermaterial wird dann nach Zusatz des zu spaltenden Substrates die spezifische Aktivität bestimmt, beispielsweise durch automatische Titration.

Die erfindungsgemäßen neuen Polymerisate zeigen folgende Vorteile:
sie lassen sich aus billigen, handelsüblichen Ausgangsprodukten herstellen; bei der Suspensionpolymerisation kann mit Wasser als Suspensionsmitel gearbeitet werden; Kohlenwasserstoffe und Chlorkoh lenwasserstoffe, wie sie bei der inversen Suspensionspolymerisation erfordlich sind, werden vermieden.

Die perlförmigen Polymerisate haben eine sehr gute Bindefähigkeit für biologisch aktive Substanzen.

Beispiele

1)-11) In einem Rundkolben mit Rührer, Thermometer, Stickstoffeinleitungsrohr und Rückflußkühler wurden 200 ml entmineralisiertes Wasser, 3,2 g Dinatriumhydrogenphosphat und 2,0 g Polyvinylpyrrolidon vom Molgewicht 360 000 vorgelegt und das Gemisch bis zur vollständigen Lösung des Polyvinylpyrrolidons ca. 20 Minuten bei 25 °C gerührt. Dann erfolgte jeweils die Zugabe einer Lösung die aus den Komponenten A'), B') und gegebenenfalls C') sowie Inertmittel und 2 g Azoisobutyronitril bestand (s. Tabelle 1). Das Gemisch wurde dann unter Rühren und Stickstoffüberlagerung langsam auf eine Temperatur von 70 °C erhitzt und 8 Stunden bei dieser Temperatur gehalten. Nach dem Abkühlen des Ansatzes auf ca. 25 °C wurde das Perlpolymerisat abgesaugt, dreimal mit jeweils 1 Liter Wasser 30 Minuten lang verrührt und abgesaugt, viermal mit jeweils 1 Liter Methanol 30 Minuten lang verrührt und abgesaugt und zweimal mit jeweils 1 Liter Aceton 30 Minuten lang verrührt und abgesaugt. Das erhaltene Perlpolymerisat wurde acetonfeucht gesiebt und im Trockenschrank bei 50 °C und 0,26 bar über Nacht unter Stickstoff getrocknet. Die Ausbeuten, Korngrößenverteilung gemäß Siebanalyse und gegebenenfalls die mittleren Porendurchmesser sowie die zu deren Bestimmung erforderlichen Porenvolumina sind in der Tabelle 1 angeführt.

**Tabelle 1**

| | Beispiele 1 | 2 | 3 | 4 | 5 | 6 | 7 | 8 | 9 | 10 | 11 |
|---|---|---|---|---|---|---|---|---|---|---|---|
| Komp. A'. [g] | | | | | | | | | | | |
| Glycidylmethacrylat | 50 | 50 | 50 | 50 | 10 | 40 | 40 | 45 | - | - | 30 |
| Allylglycidylether | - | - | - | - | - | - | - | 5 | 5 | - | - |
| Vinylglycidylether | - | - | - | - | - | - | - | - | - | 50 | - |
| Komp. B': [g] | | | | | | | | | | | |
| N,N'-Divinylethylenharnstoff | 50 | 50 | 50 | 50 | 90 | 50 | 50 | 50 | 95 | 50 | 50 |
| Komp. C': [g] | | | | | | | | | | | |
| Vinylacetat | - | - | - | - | - | 10 | - | - | - | - | - |
| Methylmethacrylat | - | - | - | - | - | - | 10 | - | - | - | - |
| Styrol | - | - | - | - | - | - | - | - | - | - | 20 |
| Inertmittel: [g] | | | | | | | | | | | |
| Cyclohexanol | 108 | 108 | - | - | 162 | 108 | 108 | 108 | 162 | 108 | 108 |
| Laurylalkohol | 12 | - | - | - | 18 | 12 | 12 | 12 | 18 | 12 | 12 |
| 2-Ethylhexylalkohol | - | 12 | - | - | - | - | - | - | - | - | - |
| n-Pentanol | - | - | 120 | - | - | - | - | - | - | - | - |
| TCD-Alkohol M | - | - | - | 120 | - | - | - | - | - | - | - |
| Ausbeute [g = % d.Th.] | 92,5 | 96,1 | 93,5 | 90,6 | 97,1 | 82,2 | 96,3 | 88,8 | 89,7 | 86,4 | 70,6 |
| Korngröße: > 300 µm [%] | - | - | - | 10 | - | - | - | - | - | - | - |
| 200-300 µm | - | 10,9 | 3,1 | 44,3 | 3,7 | 1,2 | 4,4 | - | - | 9,9 | - |
| 100-200 µm | 89,7 | 81,4 | 65,6 | 41,3 | 74,5 | 77,0 | 81,5 | 80,2 | 83,2 | 69,2 | 77,8 |
| 50-100 µm | 9,2 | 6,6 | 29,1 | 4,0 | 19,1 | 21,5 | 12,8 | 19,1 | 16,3 | 19,4 | 22,2 |
| < 50 µm | 1,1 | 1,1 | 2,6 | 0,4 | 2,8 | 0,2 | 1,3 | 0,7 | 0,5 | 1,5 | - |
| spez. Porenvolumen [$cm^3/g$] | 1,52 | - | - | 1,11 | - | - | - | 1,08 | - | - | - |
| Porendurchmesser [nm] | 60 | - | - | 35 | - | - | - | 41 | - | - | - |

12) bis 18) Zu 0,2 g eines nach einem der Beispiele hergestellten Trägermaterials wurde die Lösung einer biologisch aktiven Substanz, die 1,5-molar an Kaliumphosphat (Puffer) war einen pH-Wert von 7,6 aufwies, gegeben (Pufferlösungen in Beispiel 17 1-molar an Kaliumphosphat und 1,6 x $10^{-2}$ molar an Benzamidin, pH 7,8; in Beispiel 18 1-molar an Kaliumphosphat, pH 8). Nach 72 Stunden Fixieren bei 23 °C (Beispiel 18: Fixierzeit 16 Stunden) wurden die Perlen mit 1-molarer Kochsalzlösung und mit Pufferlösung gründlich gewaschen. Die Ausbeute an nutschenfeuchtem Material, gemessen mittels Titrierautomat bei 37 °C und einem pH-Wert von 7,8 mit penicillinsaurem Kalium als Substrat (Beispiel 17: Substrat N′-Benzoyl-L-argininethylesterhydrochlorid (BAEE) , pH 8,1; Beispiel 18: Substrat Harnstoff, pH 6,1, Temperatur 30 °C), das entsprechende Trockengewicht sowie die nach Bilanzierung von Ausgangs- und Waschwasseraktivität ermittelte Fixierausbeute (= Verhältnis Aktivität auf Träger zu angebotener Aktivität) und der η-Wert (η = gefundene Aktivität/angebotene Aktivität minus Waschwasser-Aktivität) sind in Tabelle 2 angeführt. Die Aktivität (U) ist die Umwandlung von 1 µmol Substanz pro Minute, die spezifische Aktivität =

$$\frac{\text{Umwandlung von 1 µmol Substanz}}{\text{Minute x Gramm}}$$

Tabelle 2

| | Beispiele 12 | 13 | 14 | 15 | 16 | 17 | 18 |
|---|---|---|---|---|---|---|---|
| Trägermaterial gem. Beispiel | 1 | 3 | 6 | 7 | 8 | 3 | 6 |
| Biol. aktive Substanz (Lösung) [µl] | | | | | | | |
| Penicillin-Acylase | 1200 | 1200 | 1200 | 1200 | 1200 | - | - |
| Trypsin | - | - | - | - | - | 1200 | - |
| Urease | - | - | - | - | - | - | 1200 |
| enthaltend [mg/ml] | 30 | 30 | 30 | 30 | 30 | 6,25 | 30 |
| entspr. [U/ml] | 236 | 228 | 220 | 235 | 230 | 392 | 52 |
| Ausbeute [mg] (nutschenfeucht) | 576 | 694 | 643 | 488 | 522 | 549 | 549 |
| entspr. [U/g] | 374 | 253 | 292 | 232 | 332 | 340 | 81 |
| bez. auf Trockengewicht [U/g] | 1075 | 880 | 940 | 565 | 865 | 935 | 223 |
| Fixierausbeute [%] | 76 | 64 | 71 | 40 | 63 | 40 | 66 |
| η-Wert | 0,89 | 0,70 | 0,74 | 0,74 | 0,66 | 0,52 | 0,75 |

19) Zu 0,1 g eines nach Beispiel 8 hergestellten Trägermaterials wurden 0,5 ml Carboxypeptidase B mit 310 Units/ml in 1 M Kaliumphosphatpuffer, pH 9,0, zugegeben und das Gemisch bei 16 °C 3 Tage verschlossen aufbewahrt. Danach wurden die Perlen mit 1 M Natriumchloridlösung gewaschen und in 50 mM Kaliumphosphatpuffer, pH 7,0, mit 0,02 % Natriumazid bei 4 °C aufbewahrt. Die Bindungsausbeute betrug 48 %, die Effizienz η = 0,48. Die Aktivität betrug 230 Units pro Gramm Feuchtgewicht entsprechend 710 Units pro Gramm Trockenmassen, gemessen an Hippuryl-L-Arginin als Substrat.

Vergleichsbeispiel (Nacharbeitung von EP-A 0 146 329, Beispiel 2)

Eine wässrige Phase, bestehend aus 490 ml deionisiertem Wasser, 16,2 g Natriumchlorid, 10,5 g einer 12,5 %igen Lösung von Natriumpolyacrylat und 0,9 g Pharmagelatine, gelöst in 50 ml deionisiertem Wasser, wurde in einem Reaktionsgefäß 10 min gerührt. Eine organische Phase, bestehend aus 111,4 g Trimethylolpropyltrimethacrylat, 28 g Glycidylmethacrylat, 314 g Toluol und 1,35 g Azoisobutyronitril wurde in das Reaktionsgefäß gegeben und der Ansatz 15 Minuten mit 200 Upm gerührt. Sodann wurde die Temperatur auf 65 °C erhöht und 20 Stunden auf dieser Höhe gehalten. Danach konnte der Ansatz abkühlen. Die entstandenen weißen Perlen wurden dreimal mit je 1000 ml deionisiertem Wasser und einmal mit 500 ml Toluol gewaschen; die Perlen wurden sodann im Vakuum getrocknet. Die Ausbeute an Perlpolymerisat betrug 93,5 % der Theorie. Die Siebanalyse ergab folgende Korngrößenverteilung:
>300 µm : 5,8 %; 200-300 µm : 40,9 %; 100-200 µm : 43,5 %; 50-100 µm : 7,8 %; <50 µm : 2,0 %.

Das Perlpolymerisat wurde mit Penicillinacylase als biologisch aktive Substanz umgesetzt und die biologische Aktivität bestimmt. Dazu wurden zu 0,2 g des Perlpolymerisates 1200 µl einer Penicillin-Acylase-Lösung (30 mg/ml, 230 U/ml), die 1,5-molar an Kaliumphosphat (Puffer) war und einem pH-Wert von 7,6 aufwies, gegeben. Nach 72 Stunden Fixieren bei 20 °C wurden die Perlen mit 1-molarer Kochsalzlösung und mit Pufferlösung gründlich gewaschen. Die Ausbeute an nutschenfeuchem Material betrug 501 mg mit 148 Units/g, gemessen mittels Titrierautomat bei 37 °C und einem pH-Wert von 7,8 mit penicillinsaurem Kalium als Substrat. Bezogen auf Trockengewicht waren das 370 Units/g. Nach Bilanzierung von Ausgangs- und Waschwasseraktivität blieb eine Fixierausbeute von 27 %.

**Patentansprüche für die Vertragsstaaten BE, CH, DE, FR, GB, IT, LI, NL, SE**

1. Vernetztes Polymerisat, bestehend im wesentlichen aus A) 1 bis 70 Gew.-% Einheiten, die sich von Glycidylacrylat, Glycidylmethacrylat, Allylglycidylether und/oder Vinylglycidyleter ableiten und B) 99 bis 30 Gew.-% Einheiten, die sich von N,N'-Divinylethylenharnstoff und/oder N,N'-Divinylpropylenharnestoff ableiten, wobei die Summer der Einheiten stets 100 Gew.-% ist und wobei die Polymerisatteilchen eine im wesentlichen kugelförmige Gestalt, eine mittlere Teilchengröße von 10 bis 600 µm und einen mittleren Porendurchmesser von 5 bis 1000 nm aufweisen.

2. Polymerisat nach Anspruch 1, dadurch gekennzeichnet, daß Glycidylmethacrylat eingesetzt wird.

3. Polymerisat nach Anspruch 1 oder 2, dadurch gekennzeichnet, daß es zusätzlich C) 0,1 bis 20 Gew.-%, bezogen auf das Gesamtpolymerisat, Einheiten enthält, welche sich von Vinylacetat, Methylmethacrylat, Butylacrylat und/oder Styrol ableiten.

4. Verfahren zur Herstellung eines vernetzten Polymerisats durch Copolymerisation von Monomeren in einem flüssigen Dispersionsmittel, welches unter den Polymerisationsbedingungen die Monomeren und das Polymerisat nicht löst, in Gegenwart eines radikalisch wirksamen Initiators und weiterer Hilfsstoffe sowie eines Stoffes, welcher sich in den Monomeren gut löst oder mit ihnen mischbar ist und im Dispersionsmittel praktisch unlöslich ist (Inertmittel), dadurch gekennzeichnet, daß A') 1 bis 70 Gew.-% Glycidylacrylat, Glycidylmethacrylat, Allylglycidylether und/oder Vinylglycidylether und B') 99 bis 30 Gew.-% N,N'-Divinylethylenharnstoff und/oder N,N'-Divinylpropylenharnstoff, wobei die Summe der Monomeren stets 100 Gew.-% ist, in Gegenwart von 50 bis 300 Gew.-%, Inertmittel bezogen auf die Summe der Monomeren, copolymerisiert werden.

5. Verfahren nach Anspruch 4, dadurch gekennzeichnet, daß zusätzlich die Komponent C') 0,2 bis 20 Gew.-%, bezogen auf das Monomerengemisch, Vinylacetat, Methylmethyacrylat, Butylacrylat und/oder Styrol copolymerisiert werden.

6. Verfahren nach einem oder mehreren der Ansprüche 4 und 5, dadurch gekennzeichnet, daß die Copolymerisation als Suspensionspolymerisation in Wasser als Suspensionsmittel bei einer Temperatur von 20 bis 120 °C durchgeführt wird.

7. Verwendung der Polymerisate nach Anspruch 1 als Trägermaterialien für die Immobilisierung biologisch aktiver Substanzen.

8. Verwendung nach Anspruch 7, dadurch gekennzeichnet, daß die biologisch aktiven Substanzen Enzyme sind.

**Patentansprüche für den Vertragsstaat: AT**

1. Verfahren zur Herstellung eines vernetzten Polymerisats durch Copolymerisation von Monomeren in einem flüssigen Dispersionsmittel, welches unter den Polymerisationsbedingungen die Monomeren und das Polymerisat nicht löst, in Gegenwart eines radiakalisch wirksamen Initiators und weiterer Hilfsstoffe sowie eines Stoffes, welches sich in den Monomeren gut löst oder mit ihnen mischbar ist und im Dispersionsmittel praktisch unlöslich ist (Inertmittel), dadurch gekennzeichnet, daß A') 1 bis 70 Gew.-% Glycidylacrylat, Glycidylmethacrylat, Allylglycidylether und/oder Vinylglycidylether und B') 99 bis 30 Gew.-% N,N'-Divinylethylenharnstoff und/oder N,N'-Divinylpropylenharnstoff, wobei die Summe der Monomeren stets 100 Gew.-% ist, in Gegenwart von 50 bis 300 Gew-%, Inertmittel bezogen auf die Summe der Monomeren, copolymerisiert werden.

2. Verfahren nach Anspruch 1, dadurch gekennzeichnet, daß Glycidylmethacrylat eingesetzt wird.

3. Verfahren nach Anspruch 1 oder 2, dadurch gekennzeichnet, daß zusätzlich die Komponente C') 0,1 bis 20 Gew.-%, bezogen auf das Monomerengemisch, Vinylacetat, Methylmethacrylat, Butylacrylat und/oder Stryol copolymerisiert werden.

4. Verfahren nach einem oder mehreren der Ansprüche 1 bis 3, dadurch gekennzeichnet, daß die Copolymerisation als Suspensionspolymerisation in Wasser als Suspensionsmittel bei einer Temperatur von 20 bis 120 °C durchgeführt wird.

5. Verwendung der Polymerisate nach Anspruch 1 als Trägermaterialien für die Immobilisierung biologisch aktiver Substanzen.

6. Verwendung nach Anspruch 5, dadurch gekennzeichnet, daß die biologisch aktiven Substanzen Enzyme sind.

**Claims for the Contracting State AT**

1. A process for the preparation of a crosslinked polymer by copolymerization of monomers in a liquid dispersant which, under the polymerization conditions, does not dissolve the monomers and the polymer, in the presence of a free radical initiator and other auxiliaries, and of a substance which is readily soluble in or is miscible with the monomers and is virtually insoluble in the dispersant (inert agent), which comprises copolymerization of A') 1 to 70% by weight of glycidyl acrylate, glycidyl methacrylate, allyl glycidyl ether and/or vinyl glycidyl ether and B') 99 to 30% by weight of N, N'-divinylethyleneurea and/or N, N'-divinylpropyleneurea, the total of monomers always being 100% by weight, in the presence of 50 to 300% by weight, based on the total of the monomers, of inert agent.

2. The process as claimed in claim 1, wherein glycidyl methacrylate is used.

3. The process as claimed in claim 1 or 2, wherein additionally the component C') 0.1 to 20% by weight, based on the monomer mixture, of vinyl acetate, methyl methacrylate, butyl acrylate and/or styrene is copolymerized.

4. The process as claimed in one or more of claims 1 to 3, wherein the copolymerization is carried out as suspension polymerization in water as suspending agent at a temperature of 20 to 120°C.

5. The use of the polymers as claimed in claim 1 as carrier materials for the immobilization of biologically active substances.

6. The use as claimed in claim 5, wherein the biologically active substances are enzymes.

**Claims for the Contracting States BE, CH, DE, FR, GB, IT, LI, NL, SE**

1. A crosslinked polymer which is substantially composed of A) 1 to 70% by weight of units derived from glycidyl acrylate, glycidyl methacrylate, allyl glycidyl ether and/or vinyl glycidyl ether, and B) 99 to 30% by weight of units derived from N, N'-divinylethylene urea and/or N, N'-divinylpropyleneurea, with the total of the units always being 100% by weight and with the polymer particles having an essentially spherical shape, a mean particle size of 10 to 600 µm and a mean pore diameter of 5 to 1 000 nm.

2. A polymer as claimed in claim 1, wherein glycidyl methacrylate is used.

3. A polymer as claimed in claim 1 or 2, which additionally contains C) 0.1 to 20% by weight, based on the total polymer, of units which are derived from vinyl acetate, methyl methacrylate, butyl acrylate and/or styrene.

4. A process for the preparation of a crosslinked polymer by copolymerization of monomers in a liquid dispersant which, under the polymerization conditions, does not dissolve the monomers and the polymer, in the presence of a free radical initiator and other auxiliaries, and of a substance which is readily soluble in or is miscible with the monomers and is virtually insoluble in the dispersant (inert agent), which comprises copolymerization of A') 1 to 70% by weight of glycidyl acrylate, glycidyl methacrylate, allyl glycidyl ether and/or vinyl glycidyl ether and B') 99 to 30% by weight of N, N'-divinylethyleneurea and/or N, N'-divinylpropyleneurea, the total of monomers always being 100% by weight, in the presence of 50 to 300% by weight, based on the total of the monomers, of inert agent.

5. The process as claimed in claim 4, wherein additionally the component C') 0.1 to 20% by weight, based on the monomer mixture, of vinyl acetate, methyl methacrylate, butyl acrylate and/or styrene is copolymerized.

6. The process as claimed in claim 4 or 5, wherein the copolymerization is carried out as suspension polymerization in water as suspending agent at a temperature of 20 to 120°C.

7. The use of the polymers as claimed in claim 1 as carrier materials for the immobilization of biologically active substances.

8. The use as claimed in claim 7, wherein the biologically active substances are enzymes.

**Revendications pour l'Etat Contractant AT**

1. Procédé de préparation de polymères réticulés par copolymérisation de monomères dans un milieu de dispersion liquide qui ne dissout, dans les conditions de la polymérisation, ni les monomères ni les polymères, en présence d'un inducteur radicalaire et d'autres adjuvants ainsi que d'une matière qui se dissout bien dans les monomères ou qui leur est miscible mais qui est pratiquement insoluble dans le milieu de dispersion (matière dite inerte), procédé caractérisé en ce que l'on copolymérise A') de 1 à 70 % en poids d'acrylate de glycidyle, de méthacrylate de glycidyle, d'éther allylglycidylique et/ou d'éther vinylglycidylique, la somme des proportions des monomères étant toujours égale à 100 % en poids, et B') de 99 à 30 % en poids de N,N'-divinyléthylène-urée et/ou de N,N'-divinylpropylène-urée, en présence de 50 à 300 % en poids de la matière inerte par rapport à la somme des monomères.

2. Procédé selon la revendication 1, caractérisé en ce que l'on copolymérise du méthacrylate de glycidyle.

3. Procédé selon la revendication 1 ou 2, caractérisé en ce que l'on copolymérise également, comme composante C'), de 0,1 à 20 % en poids, par rapport au mélange des monomères, d'acétate de vinyle, de méthacrylate de méthyle, d'acrylate de butyle et/ou de styrène.

4. Procédé selon une ou plusieurs des revendications 1 à 3, caractérisé en ce que l'on effectue la copolymérisation en tant que polymérisation en suspension dans de l'eau comme milieu de suspension, à une température de 20 à 120° C.

5. Emploi des polymères de la revendication 1 comme matières servant de supports pour immobiliser des substances ayant une activité biologique.

6. Emploi selon la revendication 5 dans lequel les substances à activité biologique sont des enzymes.

**Revendications por les Etats Contractants BE, CH, DE, FR, GB, IT, LI, NL, SE**

1. Polymères réticulés qui sont essentiellement formés de 1 à 70 % en poids de motifs d'acrylate de glycidyle, de méthacrylate de glycidyle, d'éther allylglycidylique et/ou d'éther vinylglycidylique, et B) de 99 à 30 % en poids de motifs de N,N'-divinyléthylène-urée et/ou de N,N'-divinylpropylène-urée, la somme de tous les motifs faisant toujours 100 %, et les particules du polymère ayant une forme essentiellement sphérique, une dimension moyenne de 10 à 600 µm et un diamètre moyen de leurs pores de 5 à 1000 nm.

2. Polymère selon la revendication 1, caractérisé en ce qu'il comprend du méthacrylate de glycidyle.

3. Polymère selon la revendication 1 ou 2, caractérisé en ce qu'il comprend en plus C) de 0,1 à 20 %, de son poids total, de motifs d'acétate de vinyle, de méthacrylate de méthyle, d'acrylate de butyle et/ou de styrène.

4. Procédé de préparation de polymères réticulés par copolymérisation de monomères dans un milieu de dispersion liquide qui, dans les conditions de la polymérisation, ne dissout ni les monomères ni les polymères formé en présence d'un inducteur radicalaire et d'autres adjuvants ainsi que d'une matière qui se dissout bien dans les monomères ou qui leur est miscible, mais qui est pratiquement insoluble dans le milieu de dispersion (matière dite inerte), procédé caractérisé en ce que l'on copolymérise A') de 1 à 70 % en poids d'acrylate de glycidyle, de méthacrylate de glycidyle, d'éther allylglycidylique et/ou d'éther vinylglycidylique, et B') de 99 à 30 % en poids de N,N'-divinyléthylène-urée et/ou de N,N'-divinylpropylène-urée, la somme des monomères étant toujours de 100 % en poids, en présence de 50 à 300 % en poids de la matière inerte par rapport à la somme des monomères.

5. Procédé selon la revendication 4, caractérisé en ce que l'on copolymérise également, comme composante C', de 0,1 à 20 % en poids, par rapport au mélange des monomères, d'acétate de vinyle, de méthacrylate de méthyle, d'acrylate de butyle et/ou de styrène.

6. Procédé selon la revendication 4 ou 5, caractérisé en ce que l'on effectue la copolymérisation par polymérisation en suspension dans de l'eau comme milieu de suspension, à une température de 20 à 120° C.

7. Emploi des polymères selon la revendication 1 comme matières servant de supports pour immobiliser des substances ayant une activité biologique.

8. Emploi selon la revendication 7, caractérisé en ce que les substances à activité biologique sont des enzymes.